(19) Europäisches Patentamt
European Patent Office
Office européen des brevets

(11) Veröffentlichungsnummer: **0 075 771**
**B1**

(12) # EUROPÄISCHE PATENTSCHRIFT

(45) Veröffentlichungstag der Patentschrift: **09.04.86**

(51) Int. Cl.⁴: **C 07 C 39/17**, C 08 G 8/30, C 08 G 61/02, C 08 K 5/00, C 08 L 21/00, C 08 L 77/00, C 07 C 37/11

(21) Anmeldenummer: **82108391.2**

(22) Anmeldetag: **11.09.82**

(54) **Neue Phenole, Verfahren zu ihrer Herstellung und ihre Verwendung als Stabilisatoren für Polymere.**

(30) Priorität: **25.09.81 DE 3138180**

(43) Veröffentlichungstag der Anmeldung: **06.04.83 Patentblatt 83/14**

(45) Bekanntmachung des Hinweises auf die Patenterteilung: **09.04.86 Patentblatt 86/15**

(84) Benannte Vertragsstaaten: **BE CH DE FR GB IT LI NL**

(56) Entgegenhaltungen:
**EP-A-0 003 338**
**BE-A-670 691**
**FR-A-1 323 858**
**FR-A-2 248 309**
**FR-A-2 307 836**
**GB-A-477 954**
**US-A-2 471 455**
**US-A-2 811 564**
**US-A-3 383 362**
**US-A-3 644 537**
**US-A-4 250 076**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Brassat, Bert, Dr., Bethelstrasse 24, D-4150 Krefeld (DE)**
Erfinder: **Buysch, Hans-Josef, Dr., Brandenburger Strasse 28, D-4150 Krefeld (DE)**
Erfinder: **Matner, Martin, Dr., Dorfstrasse 14, D-5068 Odenthal (DE)**
Erfinder: **Striegler, Hellmut, Dr., Friedrich-Bayer-Strasse 12, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 075 771 B1

LIBER, STOCKHOLM 1986

**Beschreibung**

Die Erfindung hat neue Phenole der allgemeinen Formel I

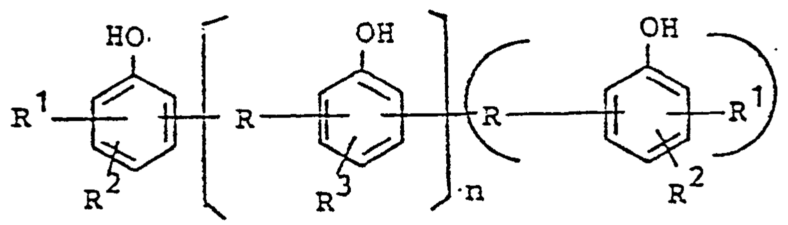 (I)

zum Gegenstand,
in der R ein Limonen-(Dipenten) -Rest oder bezogen auf das gesamte R, bis zu 60 Mol% eines Restes, abgeleitet von Divinylbenzol oder Diisopropenylbenzol, ist
$R^1$ und $R^2$ tert.-Butyl, oder Benzyl sind mit der Maßgabe, daß $R^1$ und $R^2$ nicht gleichzeitig H sein können; aber gleichzeitig $CH_3$ sein müssen, $R^3$ die gleiche Bedeutung wie $R^1$ oder $R^2$ hat oder der Rest in der runden Klammer sein kann und n eine ganze Zahl von 0 bis 20 darstellt. Verfahren zur Herstellung der neuen Phenole bestehen darin, daß man Phenol mit Limonen in Gegenwart saurer Katalysatoren und dann das erhaltene Reaktionsprodukt mit einem oder mehreren den Resten $R^1$ und $R^2$ entsprechenden Alkylierungsmitteln umsetzt, oder daß man Phenole der Formel VI

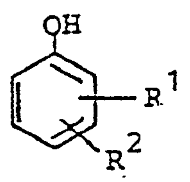 VI

in der $R^1$ und $R^2$ die oben definierte Bedeutung haben, in Gegenwart saurer Katalysatoren mit Limonen der oben beschriebenen Struktur umsetz. Die Erfindung betrifft schließlich auch die Verwendung der Phenole der allgemeine Formel I, in der zusätzlich zu den in den Ansprüchen 1 und 2 genannten Bedeutungen noch folgende auftreten können: $R^1$ und $R^2$ H, $CH^3$, Styryl, mit der maßgabe, daß nicht beide H sein können, aber beide $CH^3$ sein müssen, als Stabilisatoren für Polymere, insbesondere zur Stabilisierung von Pylymeren gegen oxidativen Abbau. Es ist bekannt, Phenol mit Pinen zu Bisphenolen umzusetzen (BE 670 691). Diese besitzen jedoch praktisch keine Wirkung als Stabilisator.

Ferner sind in der GB-pS 961 504 Umsetzungsprodukte von Terpenen mit Bisphenolen der Struktur IV und V

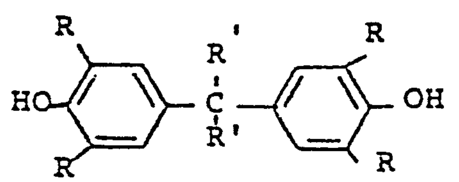

IV

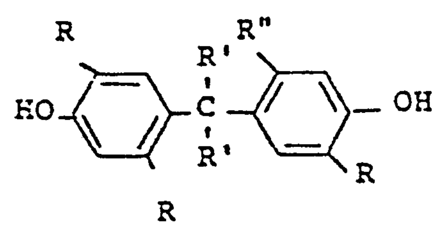

V

in denen R H oder eine Alkylgruppe mit 3 - 12 C-Atomen, $R^1$ H oder eine Alkylgruppe mit 1 - 5 C-Atomen oder eine Phenylgruppe und $R^2$ H oder eine Alkylgruppe mit 1 - 5 C-Atomen darstellt, als Stabilisatoren beschrieben, die aber eine wesentlich geringere Wirksamkeit als die er-findungsgemäßen aufweisen.

Stabilisatoren, die den erfindungsgemäßen in Struktur und Synthese am nächsten stehen, sind solche, die aus Phenolen, Dicyclopentadien und Isobutylen hergestellt werden(DE-PS 1 495 985). Diese stellen gute Stabilisatoren dar, befriedigen aber doch insichtlich antioxidativer Wirksamkeit, Verfärbungsverhalten im Licht und Extraktionsbeständigkeit nicht (vergl. Beispiel 1). Weiterhin sind strukturell ähnliche Phenole zur Stabilisierung von Polymeren bekannt. Z.B aus US-A-2 811 564, Fr-A-1323 858, US-A-3 383 362, US-A-2 471 455, Fr-A-2 248 309, 2 307 836, BE-A-670 691.

Außerdem ist man gezwungen, von dem verhältnismäßig teuren p-Kresol auszugehen, wenn man die besten Stabilisatoren dieser Reihe herstellen will (vergl. Beispiel 2).

Die erfindungsgemäßen Phenole übertreffen die Stabilisatoren entsprechend dem Stand der Technik sowohl in der antioxidativen Wirksamkeit, der geringeren Verfärbung bei Belichtung und Temperaturbelastung und Langzeitwirkung infolge verminderter Auswanderung und Extrahierbarkeit aus dem Polymersubstrat. Die Verwendung der neuen Phenole als Stabilisatoren für Polymere ist daher ein weiterer Gegenstand der vorliegenden Anmeldung.

Die erfindungsgemäßen Phenole können prinzipiell auf zwei Wegen hergestellt werden:

Durch säurekatalysierte Umsetzung von Alkylphenolen der Formel VI mit wenigstens einer freien o- oder p-Position,

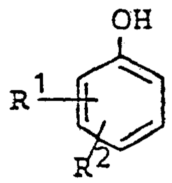

VI

in der $R^1$ und $R^2$ die oben angegebene Bedeutung haben, also auch $R^3$ sein können, mit Limonen oder durch säurekatalysierte Umsetzung von Phenol mit Limonen und anschließende Alkylierung des Reaktionsproduktes mit Isobuten oder diesem gleichwertigen Hydroxy, oder Halogenverbindungen oder mit Benzylverbindungen.

Das Molverhältnis von Phenolen zu Limonen beträgt zwischen 20/1 bis 0,5/1, vorzugsweise 15/1 bis 0,7/1, besonders aber 10/1 bis 0,8/1. Nach der Reaktion kann überschüssiges Phenol oder Alkylphenol VI durch Destillation zurückgewonnen werden, was sich bei Anwendung eines großen Überschusses empfiehlt, es kann aber auch besonders bei Anwendung eines geringen Überschusses im Reaktionsprodukt verbleiben. Wenn phenol selbst umgesetzt wird, folgt die Alkylierung mit den oben genannten Alkylierungsmitteln. Die Menge an Alkylierungsmittel entspricht den alkylierbaren Positionen in den Phenolkernen des Reaktionsproduktes von Phenol mit Limonen und beträgt im allgemeinen 0,2 bis 4 Mol, besonders 0,5 bis 3,5, besonders bevorzugt 0,5 - 3 Mol pro Mol umgesetztes Limonen.

Die Reaktionstemperatur bei beiden Reaktionsschritten liegt um 20 - 200°C, vorzugsweise 50 - 180°C, besonders bevorzugt 70 - 150°C.

Soll außer dem Limonenrest bis zu 60 Mol % besonders 50 Mol % bezogen auf das gesamte R, des Diethylbenzolrestes

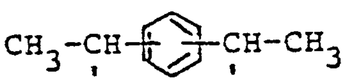 oder

des Diisopropylbenzolrestes 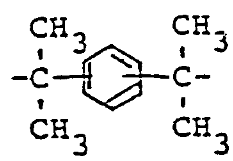

eingsführt werden, so kann dies vor oder nach der Umsetzung des Phenols mit Limonen unter den gleichen Bedingungen in Gegenwart der genannten sauren Katalysatoren, jedoch vor der Alkylierung erfolgen.

Die Herstellung von neuen Phenolen ist Gegenstand des vorliegenden Anspruchs 2.

Geeignete Ausgangsprodukte zur Herstellung der er-findungsgemäßen Phenole sind Alkylphenole der Formel VI, beispielsweise tert. Butylphenol, Benzylphenol, bes. Phenol selbst, weiter Limonen (Dipenten) und schließlich Alkylierungsmittel oder diesen äquivalenten Hydroxy- oder Halogenverbindungen wie tert. Butanol, tert. Butylchlorid, Isobutylen, Benzylchlorid und Benzylalkohol, besonders bevorzugt Isobutylen.

Geeignete Substanzen zur Einführung der Reste

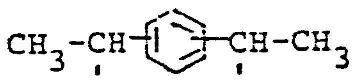 und 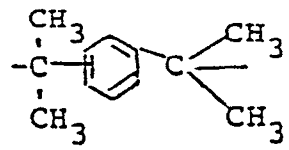

sind 1,4- und 1,3-Divinylbenzol, m- und p-Diisopropenylbenzol, α α'-Dihydroxy-m- und p-diisopropylbenzol, α,α'-Dichlor-m- und p-diisopropylbenzol,

Geeignete Katalysatoren für die Herstellung der neuen Phenole sind starke Proton- oder Lewissäuren wie Salzsäure, Schwefelsäure, Phosphorsäure, Toluolsulfonsäure, stark saure vernetzte Ionenaustauscher auf der Basis von Styrol mit Sulfonsäuregruppen, säureaktivierte Tonerden oder Borfluorid und seine Addukte wie Borfluorid-Etherar, Aluminiumchlorid, Eisen-III-chlorid, Zinkchlorid.

Diese Katalysatoren werden im allgemeinen in Mengen von 0,01 - 10 Gew.%, vorzugsweise 0,05 - 8, besonders bevorzugt 0,1 - 5 Gew%, bezogen auf das Reaktionsgemisch, eingesetzt.

Die sauren Ionenaustauscher können auch in größeren Mengen eingesetzt werden, da sie leicht z.B. durch Filtrieren abgetrennt und oft verwendet werden können.

Ähnliches trifft für die säureaktivierten Tonerden vom Bentonit- oder Montmorillonittyp zu. Die anderen Katalysatoren können durch Abdestillieren, Auswaschen mit Wasser oder Neutralisieren mit Basen entfernt oder inaktiviert werden. Die Umsetzungen können mit und ohne Lösungsmittel durchgeführt werden.

Geeignet sind unter den Reaktionsbedingungen inerte Lösungsmittel wie aliphatische und aromatische Kohlenwasserstoffe, z.B. Cyclohexan, pentan, Heptan, Ligroin, Leichtbenzin, Testbenzin, Benzol, Toluol, Xylol, Cumol, Diisopropylbenzol, Ethylbenzol, halogenierte Kohlenwasserstoffe; z.B. Methylenchlorid, Chloroform, Tetrachlorkohlenstoff, Dichlorethan, Trichlorethylen, Chlorbenzol, Brombenzol und Dichlorbenzol.

Die Phenole gemäß Anspruch 3 sind als Stabilisatoren für polymere wie Polyolefine, Polystyrole, Styrolacrylnitril-Mischpolymere, Polyurethane, Polyether, Polyester und Schmieröle geeignet.

Besondere Wirksamkeit entfalten sie jedoch in Polymeren vom Kautschuktyp, wie Polybutadienen, Polyisoprenen, Polychloroprenen, Mischpolymerisaten wie Styrolbutadienkautschuk, Nitrilkautschuk, Pfropfpolymerisaten auf Kautschukbasis wie ABS-Harzen und Polymerlegierungen mit Kautschukanteil und in Polyamiden, besonders solchen, die als Hauptbestandteil Polyamid 6 oder Polyamid 66 enthalten.

**Beispiel 1**

Zu 564 g (6 Mol) Phenol und 3 g $BF_3$ -Etherat werden unter Stickstoff und Rühren in 2 - 3 h bei 50°C 136 g 1 (1 Mol) Limonen zugetropft und das Gemisch noch 2 1/2 h bei 50°C gerührt. Überschüssiges Phenol wird bis 160°C / 9 mb im Sumpf abdestilliert. Der hellbraune Rückstand (282 g) wird in 200 ml Benzin gelöst, mit 3 g $BF_3$ -Etherat versetzt und durch Aufleiten von Isobutylen in 3 h bis zum Ende der Gasaufnahme alkyliert. Nach Eindampfen bei 150°C / 15 mb im Sumpf bleiben 371 g eines braunen spröden Harzes, das einen Sauerstoffgehalt von 6,9 % und eine osmometrisch bestimmte mittlere Molmasse von 405 aufweist.

**Beispiel 2**

Herstellung eines erfindungsgemäß zu verwendenden Phenols:

Zu einem Gemisch von 977 g (8 Mol) 2,4-Dimethylphenol und 3 g $BF_3$ -Etherat tropft man unter Rühren und Stickstoff bei 50°C in 5 h 136 g (1 Mol) Limonen und hält noch in 1 h bei 50°C. Bei 10 mb wird dann bis zu 160°C im Sumpf überschüssiges 2,4-Dimethylphenol abdestilliert. Man erhält 265 g eines gelben dickflüssigen Harzes.

**Vergleichsbeispiel A** Zu 94 g (1 Mol) Phenol in 100 ml Toluol und 5 g $BF_3$ -

Etherat tropft man unter N und Rühren bei 21 - 4°C in 5 h 150 g (1,1 Mol) Limonen in 200 ml Toluol und läßt noch 3 h rühren. Nach Stehenlassen über Nacht wird Toluol abdestilliert und der Rückstand bis 160°C / 1 mb im Sumpf von flüchtigen Verbindungen befreit. Es hinterbleiben 230 g eines gelben Harzes.

**Vergleichsbeispiel B**

Man stellt zunächst wie in Beispiel 4 ein Umsetzungsprodukt aus Phenol und Limonen her. Alkyliert dieses dann jedoch nicht mit Divinylbenzol, sondern mit Styrol.

Dazu werden 200 g des Phenol-Limonen-Umsetzungsproduktes in 200 ml Toluol gelöst, mit 3 g $B_3$ -Etherat und dann in 3 h unter $N_2$ und Rühren mit 104 g Styrol bei 25 - 8°C tropfenweise versetzt und noch 3 h rühren gelassen. Nach der Destillation flüchtiger Bestandteile bis 190°C / 18 mb im Sumpf bleiben 299 g braunes Harz.

**Beispiel 3**

Zu einem Gemisch von 1936 g (20,6 Mol) Phenol und 8 g konzentrierter Schwefelsäure tropft man bei 87 - 97°C unter Rühren und Stickstoff in 3 h 350 g (2,6 Mol) Limonen hinzu und beläßt noch 5 h bei 95°C. Man gibt 6 g NaOH-Pulver hinzu, rührt bis zu dessen klarer Lösung und destilliert bei 8 mb bis 165°C im Sumpf überschüssiges Phenol ab.

Der Sumpf (688 g) wird in Toluol gelöst, mit 4 g konzentrierter Schwefelsäure versetzt und bei 90-100°C mit Isobutylen in 7 h gesättigt. Nach Zugabe von 3 g NaOH-Pulver und einstündigem Rühren bei 90°C wird bei 20 mb bis zu einer Sumpftemperatur von 165°C flüchtiges Material abdestilliert. Man erhält 890 g eines braunen spröden Harzes.

**Beispiel 4**

Zu einem Gemisch von 1936 g (20,6.Mol) Phenol und 8 g $BF_3$-Dihydrat tropft man unter Rühren und Stickstoff bei 50°C in 6 h 350 g (2,6 Mol) Limonen und hält noch 2 h bei 50°C. Nach Stehen über Nacht destilliert man bis 165°C / 20 mb überschüssiges Phenol ab und erhält 730 g Harz als Sumpfprodukt, das in Toluol gelöst, mit 12 g $BF_3$-Etherat versetzt und bei 115°C unter kräftigem Rühren mit Isobutylen (290 g) gesättigt wird. Man rührt noch 2 h bei 90°C und destilliert bis 160°C / 20 mb im Sumpf flüchtige Bestandteile ab. Im Sumpf verbleiben 1010 g eines braunen spröden, doch klebrigen Harzes.

**Vergleichbeispiel C**

Zu einem Gemisch von 772 g Phenol und 3,2 konzentrierter Schwefelsäure tropft man unter Rühren und Rückfluß bei 80 - 90°C in 3 h 140 g Limonen, rührt noch 5 h bei 90°C, gibt 1,2 g NaOH-Pulver hinzu und destilliert bis 180°C / 10 mb im Sumpf überschüssiges Phenol ab. Man erhält 270 g braunes sprödes Harz als Sumpfprodukt.

Dieses wird nach Lösen in 300 ml Toluol und Zugabe von 3,2 g konzentrierter Schwefelsäure bei 70 - 80°C in 2 h tropfenweise mit 136 g Styrol versetzt, noch 2 h bei 70 - 80°C gerührt, mit 2,4 g NaOH-Pulver versetzt, 2 h gerührt, vom gebildeten Salz abdekantiert und bei 190°C / 16 mb im Sumpf von flüchtigen Bestandteilen befreit. Es bleiben 380 g sprödes Harz.

**Beispiel 5**

Zu einem Gemisch von 282 g (3 Mol) Phenol und 2 g konzentrierter Schwefelsäure tropft man unter Rühren und Stickstoff in 3 h bei 90 - 100°C 272 g (2 Mol) Limonen zu, hält noch 3 h bei dieser Temperatur, leitet dann bei 110 - 120°C in 18 h 240 g Isobutylen eün, gibt 1,5 g NaOH-Pulver zu und rührt noch 2 h bei 120°C. Man erhält 796 g eines braunen Weichharzes.

**Beispiel 6**

Zu einem Gemisch von 188 g (2 Mol) Phenol und 2 g konzentrierter Schwefelsäure tropft man unter Rühren und Stickstoff bei 90°C in 3 h 272 g (2 Mol) Limonen hinzu, hält noch 4 h bei 90°C und leitet dann nach Verdünnen mit 200 ml Toluol bei 100 - 110°C in 15 h Isobutylen ein. Man gibt 1,5 g NaOH-Pulver zu, rührt noch 3 h bei 100°C und destilliert dann bis 160°C / 9 mb flüchtige Bestandteile ab. Man erhält 580 g braunes sprödes Harz. Mn osmometr. = 480.

**Beispiel 7**

Zu einem Gemisch von 141 g (1,5 Mol) Phenol und 2 g p-Toluolsulfonsäure tropft man unter Rühren und Stickstoff bei 80 - 90° C in 3 h 136 g (1 Mol) Limonen, hält noch 4 h bei dieser Temperatur und leitet dann bei 110 - 112° C 7 - 8 h Isobutylen ein, rührt noch 1 h, gibt 0,5 g NaOH gelöst in 5 ml Wasser hinzu, rührt eine weitere Stunde bei 90° C und destilliert bis 120° C / 20 mb im Sumpf flüchtige Bestandteile ab. Man erhält 417 g eines gelblichen, leicht trüben, festen Harzes. Maximales Molgewicht (Gelchromatographie) 1300.

**Beispiel 8**

Zu einem Gemisch von 188 g (2 Mol) Phenol und 5 g $BF_3$-Etherat tropft man bei 80 - 90° C in 3 h unter Rühren und $N_2$ 272 g (2 Mol) Limonen, hält noch 3 h bei dieser Temperatur, verdünnt wegen der hohen Viskosität mit 200 ml Toluol und leitet bei 100° C 7 h Isobutylen bis zur Sättigung ein. Nach Abdestillieren der flüchtigen Bestandteile bis 170° C / 21 mb bleiben 650 g eines hellbraunen, spröden Harzes.

**Beispiel 9**

Aus 282 g (3 Mol) Phenol 5 g $B_3$ -Etherat und 272 g (2 Mol) Limonen stellt man wie in Beispiel 13 ein Umsetungsprodukt her, das nach Verdünnung mit 150 ml Toluol bei 90 - 100° C mit Isobutylen bis zur Sättigung Alkyliert wird. Nach Abdestillieren flüchtiger Bestandteile bis 120° C / 30 mb im Sumpf erhält man 962 g eines braunen dickflüssigen Harzes.

**Vergleichsbeispiel 1**

Analog Beispiel 1 der DE-PS 1 495 985 wird Phenol mit Dicyclopentadien und Isobutylen umgesetzt:
Zu einem Gemisch von 288 g (3,1 Mol) Phenol und 9 g $BF_3$ -Phenol-Komplex tropft man unter Rühren und Stickstoff bei 100° C 132 g (1 Mol) Dicyclopentadien in 3,5 h hinzu. Überschüssiges Phenol entfernt man durch Destillation im Vakuum bei 150° C / 5 mb, wobei 289 g als Rückstand bleiben. Diesen löst man in 290 ml Toluol, leitet nach Zugabe von 5 g $H_2SO_4$ bei 80° C 220 g Isobutylen ein und erhitzt noch 1 h. Der Katalysator wird mit Sodalösung neutralisiert, flüchtige Substanzen werden durch Destillation bis 175° C / 30 mbar und die Katalysatorreste durch Filtration entfernt. Man erhält 451 g eines braunen spröden Harzes.

**Vergleichsbeispiel 2**

Als Stabilisator wird

Wingstay L 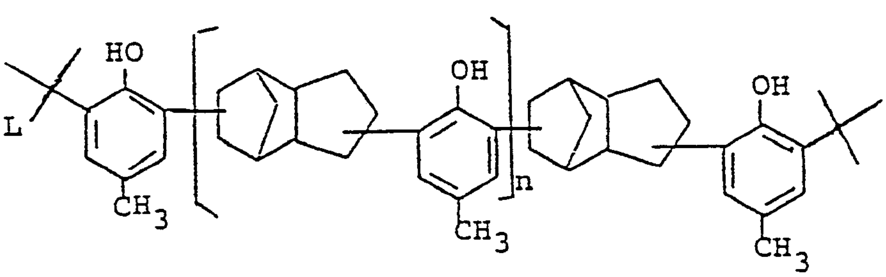

ein Produkt der Goodyear Corp. US Akron, Ohio entsprechend DE-PS 1 495 985 eingesetzt.

**Vergleichsbeispiel 3**

Als Stabilisator wird Agerite Superlite

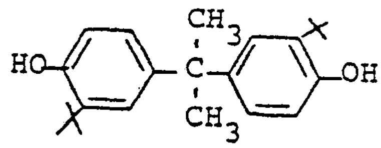

(Mischung tert. butylierter 2,2-Bis-(4-hydroxyphenol)-propane) ein Produkt der Vanderbilt Export Corp. US New York N.Y. eingesetzt.

**Vergleichsbeispiel 4**

Als Stabilisator wird das häufig in Polyamid verwendete 2,6-Di-tert.-butyl-p-kresol (Ionol) eingesetzt.

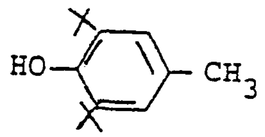

**Vergleichsbeispiel 5**

Als Stabilisator wird ein speziell für Polyamid empfohlenes Handelsprodukt der Ciba-Geigy eingesetzt.

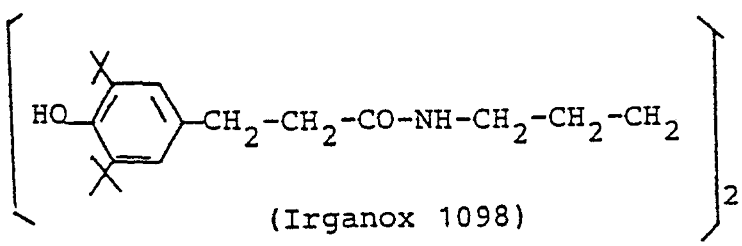

(Irganox 1098)

**Prüfung in Kautschuklatices**

Die Prüfung der Alterungsschutzmittel auf ihre Wirksamkeit wurde in Filmen vorgenommen, die aus Polymerdispersionen von selbstvernetzenden Gruppen enthaltendem Synthesekautschuk hergestellt waren.

1. Butadien/Acrylnitril-Latex der folgenden Zusammensetzung: 64 Gew.-Teile Butadien, 30 Gew.-Teile Acrylnitril, 3,5 Gew.-Teile N-Methylolacrylamid, 2,5-Gew.-Teile Acrylamid. Der Festkörpergehalt betrug 40 %.

2. Butadien/Styrol-Latex der folgenden Zusammensetzung: 54 Gew.-Teile Butadien, 37 Gew.-Teile Styrol, 3 Gewl-Teile Acrylnitril, 3 Gew.-Teile N-Methoxymethyl-methacrylamid, 3 Gew.-Teile Methacrylsäure; Festkörpergehalt 50 %.

Den Latices wurden die Alterungsschutzmittel in emulgierter Form unter Rühren zugesetzt. Die Dosierung betrug 1,2 Gew-.%, bezogen auf Festkörpergehalt. Die Emulsionen wurden hergestellt, indem die in Benzin gelösten (1 Teil Stabilisator, 2 Teile Benzin) Substanzen mittels eines Ultra-Turrax in einer 8,5-prozentigen Emulgatorlösung verwirbelt wurden. Nach Zusatz zum Latex wurde weitere 24 h gerührt, um der Emulsion Gelegenheit zur Anlagerung an die Latexpartikel zu geben (Reifeprozeß).

Die Filmbildung erfolgte durch Ausgießen des zwecks optimaler Vernetzung auf pH 2,5 (Oxalsäure) eingestellten Latex auf Tonplatten und Trocknen bei Raumtemperatur. Nach Verdampfen des Wassers wurde der Ver-netzungsprozeß durch 10 Minuten Tempern bei 150°C vollzogen.

Folgende Prüfungen wurden durchgeführt.:

1. Bestrahlung mit UV-Licht über 6 Tage. Lampe: Vitalux 300 W, Hersteller Fa. Osram. Probenabstand: 40 cm. Bewertet wurde die Verfärbung.

2. Alterung in Heißluft bei 140°C im Umluft-Trockenschrank. Bewertet wurde in den Abständen 6, 24, 48, 72 h: Verfärbung, Grad der Verhärtung, Änderung von Spannungswert und Bruchdehnung.

Zur Bewertung der Extraktionsbeständigkeit wurden die Prüflinge, die durch Ausstanzen aus den Filmen gewonnen waren, in Perchlorethylen extrahiert. Diese Maßnahme wurde bei Raumtemperatur durch Schütteln in einer geschlossenen Flasche mit einem 1000 %igem Über-schuß an Perchlorethylen während 30·Minuten durchgeführt. Die prüflinge wurden danach mit papier abgetupft und an der Luft trocknen gelassen. Wie die nicht extrahierten Prüflinge wurden sie in Heißluft bei 140°C gealtert und bewertet.

**Diskussion der Ergebnisse**

Tabelle 1 zeigt die verbesserten Eigenschaften der er-findungsgemäßen Stabilisatoren. Insbesondere in dem langsameren Abfall der Bruchdehnung wird deutlich, daß diese Substanzen das durch die Stabilisatoren nach dem Stand der Technik vorgegebene Niveau übertreffen. Bemerkenswert ist die geringere Tendenz zum Verfärben.

In Tabelle 2 ist dargestellt, inwieweit die verminderte Extrahierbarkeit der beanspruchten produkte sich auf die Alterung auswirkt. Es ist für den Fachmann deutlich erkennbar, daß die Schutzwirkung erheblich länger währt als mit den Stabilisatoren der Vergleichsbeispiele 1 und 3.

Tabelle 1: Prüfung im SBR-Latex

| Stabilisator gem. Bsp. | Spannungswerte/Bruchdehnung (MPa/%) vor und nach Alterung bei 140°C, 0-Wert | nach 6 h | 24 h | 48 h | 72 h | Verfärbung 6d UV Bel. |
|---|---|---|---|---|---|---|
| ~~(6)~~ (3) | 1,2/670 (1) | 3,8/410 (2) | 4,1/400 (2) | 4,4/400 (3) | 3,6/350 (3) | 2 |
| ~~(5)~~ (VB) | 2,0/790 (1) | 4,0/420 (2) | 4,6/420 (2) | 4,8/390 (2) | 3,3/360 (2) | 3 |
| ~~(8)~~ (VC) | 1,6/700 (1) | 2,9/440 (2) | 2,4/430 (3) | 3,0/350 (3) | 3,4/340 (3) | 3 |
| ~~(9)~~ (5) | 1,7/720 (1) | 4,4/470 (2) | 4,9/450 (3) | 3,2/350 (3) | 4,1/360 (3) | 2 |
| (V 1) | 4,8/690 (1) | 4,8/550 (2) | 4,6/360 (4) | 3,6/190 (4) (a) | 4,0/110 (5) (b) | |
| (V 2) | 4,1/600 (1) | 3,4/510 (2) | 3,5/340 (4) | 3,9/250 (5) | 4,7/120 (5) (a-b) | 3 |
| | | Prüfung im NBR-Latex | | | | |
| ~~(6)~~ (3) | 1,2/440 (1) | 5,4/390 (2) | 6,9/400 (3) | 6,0/325 (3) | 5,4/310 (3) | 2 |
| ~~(9)~~ (5) | 1,4/410 (2) | 5,4/370 (3) | 6,2/320 (3) | 5,5/310 (4) | 4,9/270 (4) | 3 |
| ~~(8)~~ (VC) | 1,4/420 (1) | 5,1/350 (2) | 5,6/310 (3) | 5,6/310 (4) | 5,4/250 (4) | 3 |
| ~~(10)~~ (6) | 2,6/470 (2) | 6,1/350 (3) | 5,4/290 (3) | 5,1/270 (4) | 4,2/230 (4) | 3 |
| (V 1) | 1,2/380 (2) | 1,9/330 (3) | 2,1/310 (3) | 2,6/240 (4) | 2,5/200 (5) (a-b) | |
| (V 2) | 2,4/450 (1) | 5,1/390 (2) | 5,2/280 (3) | 5,7/200 (4) (a) | 5,7/150 (4) (b) | 3 |

Verfärbung:
(1) farblos
(2) gering
(3) hellbraun
(4) dunkelbraun
(5) schwarz

Verhärtung:
a) Beginn der Verhärtung
b) weitgehende Verhärtung
c) Filme spöde und brüchig

(V 1): Vergleichsversuche
(V 2): Vergleichsversuche

Tabelle 2: Spannungswerte/Bruchdehnung (MPa/%) vor und nach mehrfacher Extraktion mit Perchlorethylen

Stab Alterung nach Stunden, in SBR Latex

| Stab gem. Bsp. | 0-Wert | ohne Extraktion | | | Extraktion 1 x | | | 2 x | | | 3 x | | |
|---|---|---|---|---|---|---|---|---|---|---|---|---|---|
| | 0 h | 24 h | 40 h | 72 h | 24 h | 40 h | 72 h | 24 h | 40 h | 72 h | 24 h | 40 h | 72 h |
| V 3 | 4,1/440 (1) | 4,8/330 (2) | 5,0/210 (3) | 4,4/210 (4) | 5,1/240 (4) | 0,4/0 (5)(c) | 5,4/0 (5)(c) | 2,0/160 (4) | 2,6/90 (5)(b) | 2,7/0 (5)(c) | 2,2/110 (4)(b) | 2,0/0 (5)(c) | 4,0/0 (5)(c) |
| V 1 | 4,0/690 (1) | 4,6/320 (3) | 3,8/190 (4) | 4,0/110 (4)(b) | 2,8/110 (4) (b) | 2,3/0 (5)(c) | 4,5/0 (5)(c) | | | | | | |
| 3 ø | 3,1/060 (1) | 4,1/340 (2) | 2,6/310 (3) | 3,7/280 (3) | 2,7/250 (4) | 2,7/190 (5)(a) | 5,0/50 (5)(b) | 2,7/220 (3) | 2,7/190 (5)(a) | 5,9/ 90 (5)(b ) | 2,7/220 (4) | 3,0/110 (5)(b) | 4,7/40 (5)(b-c) |
| in NBR-Latex | | | | | | | | | | | | | |
| V 3 | 3,4/360 (1) | 4,9/200 (3) | 7,1/260 (3) | 5,4/200 (4) | 5,0/310 (3) | 8,0/310 (4) | 5,6/200 (4) | 1,8/70 (5)(b) | 4,1/0 (5)(c) | 5,4/0 (5)(c) | 2,2/0 (5)(c) | 5,2/0 (5)(c) | 3,8/0 (5)(c) |
| 3 ø | 2,5/420 (1) | 6,0/320 (3) | 4,2 /300 (3) | 4,4 /270 (3) | 5,4/310 (3) | 6,0/300 (3) | 4,2/260 (4) | 5,0/390 (3) | 5,9/290 (4) | 4,0/250 (4) | 4,1/270 (3) | 3,9/200 (4) | 3,8/230 (4) |

Verfärbung:
(1) farblos
(2) gering
(3) hellbraun
(4) dunkelbraun
(5) tief dunkelbraun-schwarz

Verhärtung:
a) Beginn der Verhärtung
b) weitgehende Verhärtung
c) Filme spröde und brüchig

**Prüfung der Stabilisatorwirkung im Polyamid-6**

Zur Prüfung der Stabilisatorwirkung wurde die zu prüfende Substanz in einer Konzentration von 0,5 Gew.% durch einen Extruderdurchgang homogen in 6-Polyamid ($\eta_{rel}$ = 4,0) eingearbeitet. Das so stabilisierte Material wurde zu Normkleinstäben verspritzt und einer Wärmealterung unter Luftzutritt bei 150 ± 0,5°C unterworfen. Nach 1, 2, 4, 8 usw. Tagen wurden jeweils 8 Normkleinstäbe entnommen und nach zweistündiger Abkühlung im Exsiccator auf Schlagzähigkeit nach DIN 53 453 untersucht. Die Prüfung gilt als bestanden, wenn mindestens die Hälfte der Prüfkörper nicht gebrochen ist, oder wenn die durchschnittliche Schlagzähigkeit der gebrochenen Stäbe oberhalb 30 kJ/m² liegt. Die Bewertung der Stabilisatorwirkung geschieht nach einer Stufeneinteilung entsprechend der folgenden Tabelle:

| | | | | | |
|---|---|---|---|---|---|
| Wärmealterungszeit bis zum Nicht-Bestehen der Schlagprüfung | 1 | 2 | 4 | 8 | 16 etc. Tagen |
| Wirksamkeitsstufe des Stabilisators | 0 | 1 | 2 | 3 | 4 |

Die Prüfung führte zu folgenden Ergebnissen:

| Stabilisator 0,5 Gew.% | Bewertung |
|---|---|
| aus Beispiel 3 | |
| aus Vergleichsbeispiel 3 | 1 - 2 |
| aus Vergleichsbeispiel 4 | 2 |
| aus Vergleichsbeispiel 5 | 1 - 2 |

Das erfindungsgemäß stabilisierte Polyamid übersteht demnach eine viermal längere Alterungszeit als das nach dem Stand der Technik stabilisierte bis zum Versagen der Schlagzähigkeitsprüfung.

**Patentansprüche**

1. Phenole der allgemeinen Formel I,

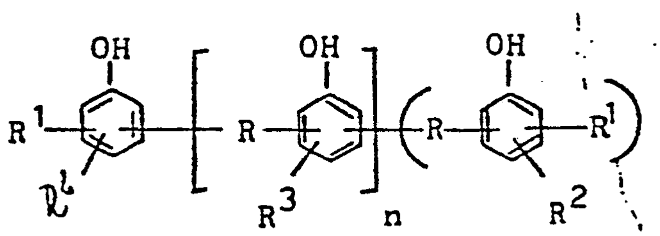

(I)

in der R ein Limonen-(Dipenten)-Rest oder, bezogen auf das gesamte R, bis zu 60 % (Mol) eines

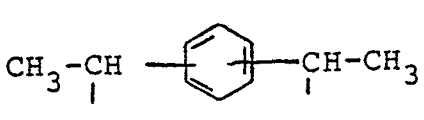 oder 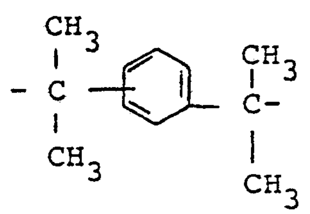 Restes ist,

$R^1$ und $R^2$ tert-Butyl oder Benzyl sind, $R^3$ die gleiche Bedeutung wie $R^1$ oder $R^2$ hat oder der Rest in der runden Klammer sein kann,

n eine ganze Zahl von 0-20 ist.

2. Phenole der allgemeinen Formel I, gemäß Anspruch 1, in der R ein Limonen-(Dipenten)-Rest ist,
$R^1$ und $R^2$ tert-Butyl sind,
$R^3$ tert-Butyl oder den Rest in der runden Klammer darstellt, dadurch hergestellt, daß man im Molverhältnis 10/1 bis 0,8/1 Phenol mit Limonen zu einem Polyphenol umsetzt in Gegenwart einer starken Protonoder Lewis-Säure bei Temperaturen von 70 bis 150°C und anschließend dieses Polyphenol mit 0,5-3 Mol Isobuten pro Mol Limonen in Gegenwart einer starken Proton- oder Lewis-Säure alkyliert.

3.) Verwendung von Phenolen der allgemeine Formel I, in der zusätzlich zu den in den Ansprüchen 1 und 2 genannten Bedeutungen noch folgende auftreten können: $R^1$ und $R^2$ H,$CH_3$, Styryl, mit der Maßgabe, daß nicht beide H sein können, aber beide $CH_3$ sein müssen, als Stabilisatoren für Polymere.

**Claims**

1. Phenols of the general formula 1

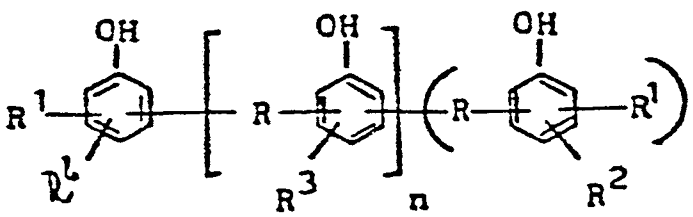

(I)

R is a limonene (dipentene) radical or, based on R as a whole, up to 60% (mol) of a

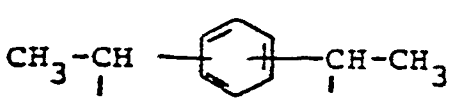

or

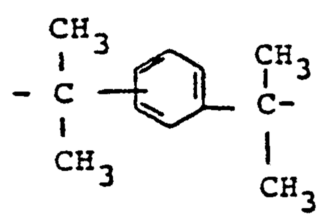

radical,

radical
$R^1$ and $R^2$ are tert-butyl or benzyl,
$R^3$ has the same meaning as $R^1$ or $R^2$ or can be the radical in the round brackets,
and
n is an integer from 0-20.

2. Phenols of the general formula I, according to Claim in which
R is a limonene (dipentene) radical,
$R^1$ and $R^2$ are tert-butyl,
$R^3$ represents tert-butyl or the radical in the round brackets,
prepared by reacting, in a molar ratio of 10/1 to 0.8/1, phenol with limonene to give a polyphenol, in the presence of a strong protonic or Lewis acid at temperatures of 70 to 150°C, and then alkylating this polyphenol with 0.5-3 mol of isobutene per mol of limonene in the presence of a strong protonic or Lewis acid..

3. Use of phenols of the general formula I, in which, in addition to the meanings given in Claims 1 and 2, the following can also arise: $R^1$ and $R^2$ - H, $CH_3$, styrene, with the proviso that $R^1$ and $R^2$ cannot both be H, but both must be $CH_3$, as stabilisers for polymers.

**Revendications**

1. Phénols de formule générale I:

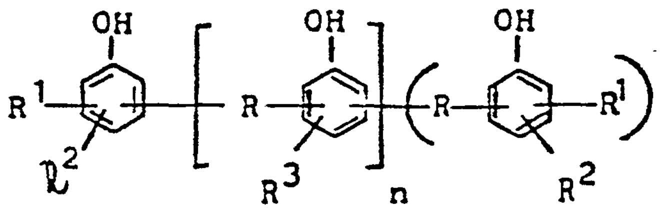

(I)

dans laquelle R est un reste de limonène-(dipentène) ou, par rapport à la totalité de R, jusqu'à 60% (en moles) d'un reste

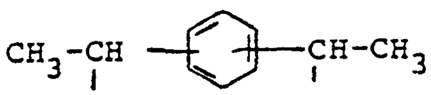

ou

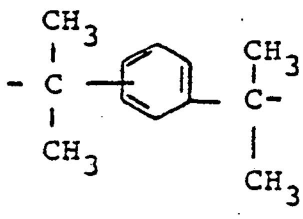

$R^1$ et $R^2$ représentent un groupe tertio-butyle ou benzyle,

$R^3$ a la même définition que $R^1$ ou $R^2$ ou peut être le reste placé entre parenthèses,

n est un nombre entier de 0 à 20.

2. Phénols de formule générale I suivant la revendication 1, dans laquelle R est un reste de limonène(dipentène),

$R^1$ et $R^2$ sont des groupes tertio-butyle,

$R^3$ est un groupe tertio-butyle ou représente le reste placé entre parenthèses, que l'on prépare en faisant réagir dans le rapport molaire de 10/1 à 0,8/1 du phénol avec du limonène pour former un polyphénol, en présence d'un acide protonique fort ou d'un acide de Lewis à des températures de 70 à 150°C, puis on alkyle ce polyphénol avec 0,5-3 moles d'isobutène par mole de limonène en présence d'un acide protonique fort ou d'un acide de Lewis.

3. Utilisation de phénols de formule générale I, dans laquelle en plus des définitions mentionnées dans les revendications 7 et 2, peuvent encore apparaître les définitions suivantes: R et R peuvent représenter H, $CH_3$, un groupe styryle, sous réserve que tous deux ne puissent pas représenter H, mais que tous deux doivent représenter $CH_3$, comme agents stabilisants pour des polymères.